# EUROPEAN PATENT APPLICATION

(11) **EP 0 642 800 A1**
(43) Date of publication of application: **15.03.1995**
(21) Application number: 94302965.2
(22) Date of filing: 25.04.1994
(51) Int. Cl.: A61M 1/00, A61B 1/12

(54) **Irrigation probe assembly**

(30) Priority: 09.09.1993 US 118562
(71) Applicant: CITATION MEDICAL CORPORATION, Reno, Nevada 89502 (US)
(72) Inventor: Middle, George H., Reno, Nevada 89509 (US); Evans, Edward A., Sparks, Nevada 89436 (US); Purdy, Craig A., Sparks, Nevada 89436 (US)
(74) Representative: Coxon, Philip

(57) **Abstract**

An irrigation probe assembly is provided which can be used during-surgery. This instrument includes a hollow handle (12) connected to a hollow probe tip (26). The probe tip includes at or near its distal end one or more openings (36) through which the irrigating fluid is drained from or released into the subject. The distal end of the probe tip may be bent to improve the instrument's ability to be manipulated after insertion into the surgical site. The proximal end (14) of the probe tip is inserted into the handle and is connected to a fluid line (18). A flow regulator (40) is included in the handle portion which, when operated, selectively restricts the flow of irrigating fluid through the instrument and out of or into the surgical site.

## Description

### FIELD OF THE INVENTION

The present invention generally relates to probes used during surgery. More particularly, the present invention relates to surgical probes used during operations requiring concurrent probing and irrigating. The present invention is particularly, although not exclusively, useful for probing and irrigating the surgical site of a patient during endoscopic surgery.

### BACKGROUND OF THE INVENTION

Modern surgical procedures attempt to minimize the invasive aspects of surgery. This is the result of the widely accepted belief that patient discomfort, overall recovery time, as well as the likelihood of post-surgical complications are generally proportional to the invasiveness of the particular procedure.

One way to reduce the invasiveness of relatively simple surgical procedures such as exploratory surgery is through the use of endoscopes. Endoscopy is a surgical procedure where a thin scope is placed into the person being examined. Some specific examples include laparoscopy, where a scope is placed into the abdominal cavity, esophascopy, where the scope is placed into the esophagus, and arthroscopy, where the scope is placed into a joint.

Using endoscopes, the surgeon is able to view the interior of the person's body without the necessity of making a large incision. Instead, the endoscope, which includes a thin, insertable probe, requires only a small incision or hole into which the probe is inserted. It is the reduced trauma at the entry point which results in the reduced recovery time and reduced post-surgical complications such as infections or ruptured suture lines. Reduced trauma results because both the skin and the musculature are only minimally disturbed.

The rapidly expanding uses for endoscopy require new types of support instruments which, like the endoscope, are minimally invasive. For example, in an arthroscopy procedure which seeks to allow a surgeon to inspect a joint, the tip of the arthroscope is placed into the joint. The tip includes an optical viewing system such as a small remote camera lens. Often, the joint to be inspected is damaged and cartilage or tissue is displaced. Further, the injury and the entry of the scope itself can cause a modicum of bleeding within the joint or the tissue surrounding the joint. This displaced cartilage and localized bleeding can obscure the lens in the arthroscope, or otherwise conceal the areas to be examined, and prevent the surgeon from viewing the joint. Therefore, localized removal of blood or tissue fragments is desirable. Finally, visibility within the joint is often obscured by surrounding tissues, so that it is desirable to be able to expand or distend the joint area to increase visibility.

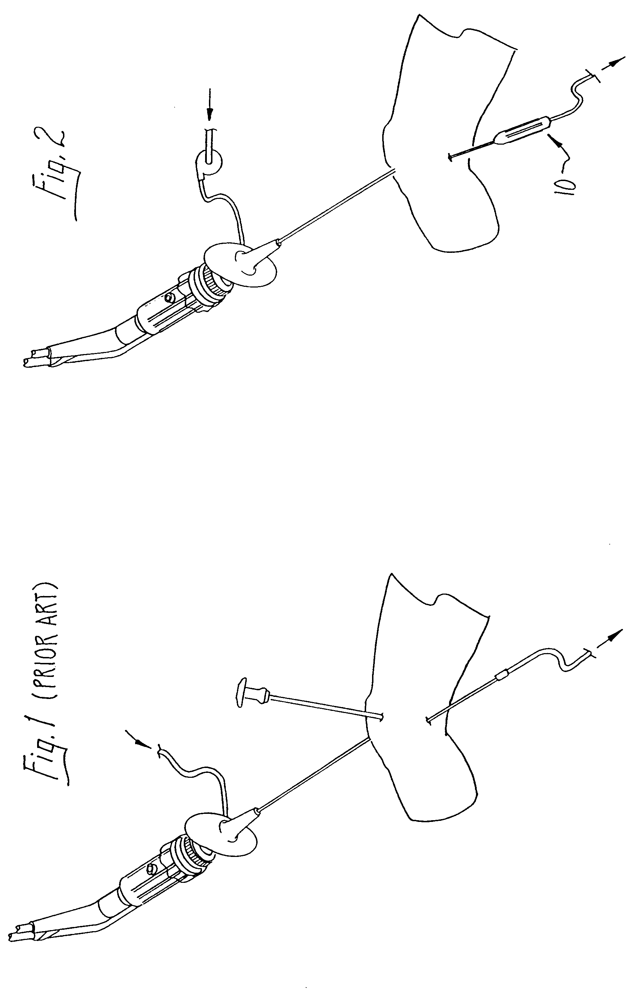

To overcome the problem of reduced visibility resulting from localized bleeding or tissue fragmentation, other tools are used in conjunction with the arthroscope. In order to remove the blood or other obstructing material from the area to be viewed, the joint is typically irrigated through a cannula surrounding the arthroscope. Irrigation fluid is typically drained through a second portal. The displaced cartilage or tissue in the joint is moved from in front of the lens using yet another tool, such as a solid probe which resembles a thin ice pick. This solid probe is normally inserted into the joint using a third opening and is manipulated by the surgeon or his assistant to clear the lens obstruction. A joint having three entry points, as commonly used in the prior art, is shown in Fig. 1.

By selective manipulation of the arthroscope, the irrigation drainage means, and the solid probe, the surgeon is able to view the joint. Unfortunately, this surgical procedure has a number of shortcomings. First, too many instruments may be involved for one person to manipulate. This requires that a second person assist the surgeon by manipulating one or more instruments according to the surgeon's instructions. Another problem associated with the foregoing three instrument arthroscopy is that typically three insertion points are necessary. This is a cause for concern because each insertion point is a separate trauma point at which infections or other complications can potentially arise. Finally, drainage of irrigation fluid as currently known may not provide for selectively localized removal of tissue fragments during lavaging of the joint.

The foregoing problems arising during arthroscopy have similar counterparts in other endoscopy procedures. In fact, most endoscopy procedures have some degree of visibility obstruction resulting from blood or tissue in front of the endoscope lens, necessitating the use of a probe and irrigation fluid.

In light of the above, it is an object of the present invention to provide a surgical instrument which minimizes the overall invasiveness of a surgical procedure. It is another object of the present invention to provide a surgical instrument which can reduce the number of surgical entry points during endoscopy. It is yet another object of the present invention to provide a single surgical instrument which can be used as both a probing tool and a tool for selectively localized drainage of irrigation fluid and tissue fragments. Still another object of the present invention is to provide an irrigation probe which allows the surgeon to probe the surgical site while regulating the flow of irrigating fluid using only one hand. Yet another object of the present invention is to provide an irrigation probe which is relatively easy to manufacture and which is comparatively economical.

### SUMMARY OF THE INVENTION

A surgical instrument is provided which can concurrently probe and irrigate a surgical site. This irrigation probe includes a handle which is an elongated member sized to allow gripping by a surgeon. Attached at the handle is a connector which allows the irrigation probe to be connected to a fluid line through which the irrigation fluid may flow.

Attached to the irrigating probe's handle, at its distal end, is an elongated and hollow probe tip which can be inserted into a surgical site. The distal end of the probe tip is tapered to facilitate insertion into the surgical site. The probe tip of the instrument includes one or more openings through which the irrigating fluid can pass. These openings in the probe tip can be at the distal end of the probe tip or along the shaft, e.g., a lateral opening, or both. The distal end of the probe tip is bent to allow the surgeon operating the instrument to more effectively manipulate the probe during surgical procedures.

The proximal end of the probe tip is attached to the distal end of the handle and is in flow communication with a connector on the handle. In combination, the connector, the handle flow control path, and the probe tip form a continuous fluid passageway between the fluid line and the surgical site.

Also included on the handle of the irrigating probe is a flow regulator. This flow regulator includes an adjustable flow restrictor which in a preferred embodiment provides a flow control button for the surgeon operating the instrument. To restrict the flow of the irrigating fluid through the instrument, the surgeon presses the flow control button.

An alternate embodiment is provided which, because of the detachability of the probe tip, probe tip connector, handle and fluid line connector, allows simplified sterilization and component reusability. Another alternate embodiment includes a valve as a flow regulator.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a perspective view of a joint which has tools inserted into three separate entry points, as is currently known in the art;
Figure 2 is a perspective view of a joint which has tools inserted into only two entry points, as is enabled through use of the irrigation probe of the present invention;
Figure 3 is a perspective view of the irrigation probe of the present invention;
Figure 4 is a cross-sectional view of the irrigation probe of the present invention;
Figure 5A is a partial cross-sectional view of the distal end of one embodiment of the tip of the irrigation probe of the present invention;
Figure 5B is a partial cross-sectional view of the distal end of an alternate embodiment of the tip of the irrigation probe of the present invention;
Figure 6A is a cut-away view of one embodiment of the flow regulator of the irrigation probe of the present invention;
Figure 6B is a cut-away view of an alternate embodiment of the flow regulator of the irrigation probe of the present invention;
Figure 7 is a partial cross-sectional view of an alternate embodiment of the irrigation probe of the present invention, which includes detachable parts;
Figure 8 is a partial cross-sectional view of the connection between the detachable probe tip and the handle of the alternate embodiment of the irrigation probe of the present invention;
Figure 9 is a partial cross-sectional view of the distal end of the handle of the alternate embodiment of the irrigation probe of the present invention;
Figure 10 is a cross-sectional view of the detachable probe tip connector of the alternate embodiment of the irrigation probe of the present invention;
Figure 11A is a cross-sectional view of a detachable probe tip of the alternate embodiment of the irrigation probe of the present invention;
Figure 11B is a cross-sectional view of an alternate embodiment of the probe tip shown in Figure 11A; and
Figure 12 is a perspective view showing an alternate embodiment having a valve to regulate flow through the handle, and showing the interchangeability of probe tips.

### DESCRIPTION OF PREFERRED EMBODIMENTS

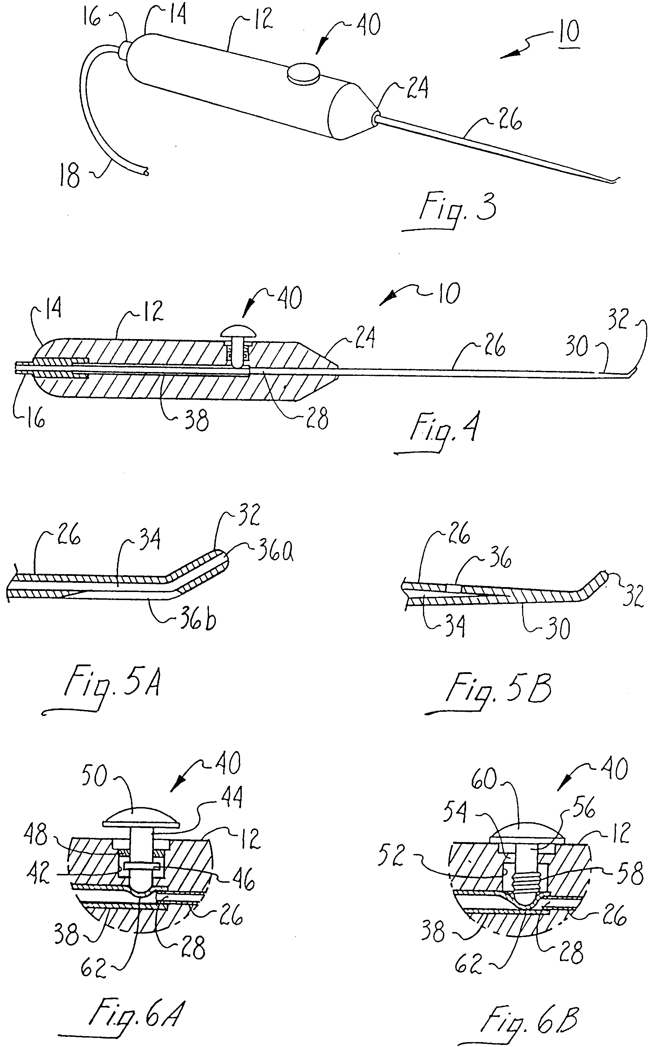

Fig. 2 shows a joint having only two entry points, as is enabled by use of the probe 10 of the present invention. A pump or other fluid movement means introduces irrigation fluid through an arthroscope cannula. Fluid flows out of the joint through probe 10. Flow can be reversed and introduced through probe 10 by connection of the pump to probe 10. Referring now to Fig. 3, the irrigation probe instrument of the present invention is shown and generally designated 10. As shown in Fig. 3, instrument 10 includes a handle 12 which is elongated and hollow and is sized to allow gripping by the surgeon operating the instrument. Handle 12 is preferably made of a hard material such as hard plastic or metal.

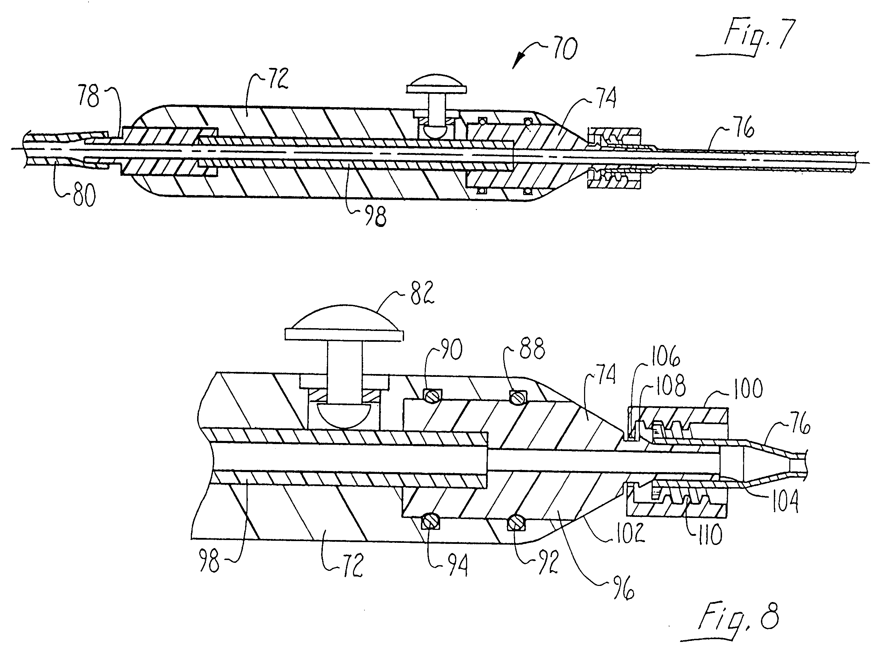

Referring now to both Fig. 3 and Fig. 4, it is shown that included at the proximal end 14 of the handle 12 is a connector 16 which allows the instrument to be connected to an irrigation fluid flow line 18. While the present invention will be described as accomplishing the drainage of fluid from the joint, one skilled in the art will appreciate that the present invention could also be used to accomplish the introduction of irrigation fluid into the joint. In such an alternate application, the fluid source may be a syringe or a pump connected to fluid line 18. As those skilled in the art will also appreciate, connector 16 can be any of a number of the many well known connectors such as a Luer Lock connector. Preferably, connector 16 will allow easy, but secure connection, and will not significantly restrict the surgeon during surgical procedures.

Attached at the distal end 24 of the handle 12 of the instrument 10 is an elongated and hollow probe tip 26 which is insertable into a surgical site. Probe tip 26 is preferably made of a hard material such as hard plastic or metal such as stainless steel. The proximal end 28 of the probe tip 26 is preferably inserted a sufficient distance into handle 12 to ensure a firm and secure connection. The distal end 30 of the probe tip 26 forms an extremity 32 which is tapered to allow the probe tip 26 to enter the surgical site. Moreover, extremity 32 is bent to allow the surgeon to more effectively manipulate the instrument 10 during surgical procedures. As those skilled in the art will appreciate, while the angle of the bend in the probe shown is approximately forty-five (45) degrees, the actual bend may vary and is preferably in the range of zero to ninety (0-90) degrees.

Referring now to Fig. 5A and Fig. 5B, alternate embodiments of the distal end 30 of the probe are shown. Probe tip 26 is hollow, forming a lumen 34. Lumen 34 is in fluid communication with the exterior of probe tip 26 via one or more openings 36, 36a, 36b at or near extremity 32 of the probe tip 26. Fig. 5A shows a probe tip 26 including an opening 36a at the extremity 32 and a lateral opening 36b along the length of the probe tip 26. Fig. 5B, on the other hand, shows a tapered distal end 30 of the probe tip 26 including only one lateral opening 36.

Referring now to Fig. 4, the proximal end 28 of probe tip 26 is connected to one end of hollow flow control tube 38 which is inside the handle 12. The other end of flow control tube 38 is connected to connector 16. Flow control tube 38 is preferably made of a flexible material such as soft plastic. In combination, connector 16, flow control tube 38 and probe tip 26 form a continuous fluid passageway between the fluid line 18 and the openings 36 in the probe tip 26.

As is shown in Fig. 3 and Fig. 4, handle 12 includes a flow regulator 40. Details of the flow regulator 40 are best appreciated by referring to Fig. 6A and 6B where alternate embodiments of the flow regulator 40 are shown. Fig. 6A shows a portion of the handle 12 which includes the flow regulator 40. Also shown is the proximal end 28 of probe tip 26 which is attached to flow control tube 38 in such a manner as to ensure a fluid seal between the components. This fluid tight attachment can be by insertion of probe tip 26 into flow control tube 38 or by other means, depending upon the materials used. Also shown is a recess 42 which begins at the surface of handle 12 and extends inwardly to form a passageway through the handle to the lumen of the handle, which contains the flow control tube 38. Partially inserted into recess 42 is a flow restrictor 44 which is formed with an annular ridge 46. Restrictor 44 is retained in recess 42 by a retainer 48 fixably placed into the recess behind the ridge 46 of restrictor 44. Cooperation between the retainer 48 and the ridge 46 prevents the restrictor 44 from exiting the recess 42 while allowing slidable movement of the restrictor 44. One end of restrictor 44 is positioned to contact flow control tube 38. The other end of restrictor 44 extends outwardly from the recess 42 and has attached to it a button 50. Button 50 provides an enlarged contact surface for operation of the flow regulator 40 by the surgeon using the instrument 10.

Referring now to Fig. 6B, an alternate flow regulator 40 is shown. Like shown in Fig. 6A, Fig. 6B shows a portion of the handle 12 which includes the flow regulator 40. Also shown is the proximal end 28 of probe tip 26. Proximal end 28 is shown inserted into flow control tube 38 a distance sufficient to ensure a fluid seal between the components. Also shown is a recess 52 which begins at the surface of handle 12 and extends inwardly to form a passageway through the handle to the lumen of the handle, which contains flow control tube 38. Unlike the recess 42 shown in Fig. 6A, recess 52 includes a threaded flange 54. Partially inserted in recess 52 is a flow restrictor 56 which is formed with threads 58. Restrictor 56 is threaded into recess 52 until all of the threads 58 have passed through flange 54. Once the threads 58 have passed through the flange 54, restrictor 56 is slidably retained in recess 52 by cooperation between the flange 54 and the threads 58. One end of restrictor 56 is positioned so as to contact flow control tube 38. The other end of restrictor 56 extends outwardly from the recess 52 and has attached to it a button 60. Button 60 provides an enlarged contact surface for operation of the flow regulator 40 by the surgeon using the instrument 10.

Referring now to Figs. 7 through 11B, an alternate embodiment of the present invention is shown and generally designated 70. As is best seen in Fig. 7, instrument 70 is similar to device 10, described above, except instrument 70 includes structure which allows detachment of the various components. Detachability of components is beneficial for purposes of sterilization and reuse of the components. Generally, the detachable components of instrument 70 include a handle 72, a probe connector 74 inserted into handle 72, a probe tip 76 attached to probe connector 74, and a fluid connector 78 between handle 72 and fluid line 80.

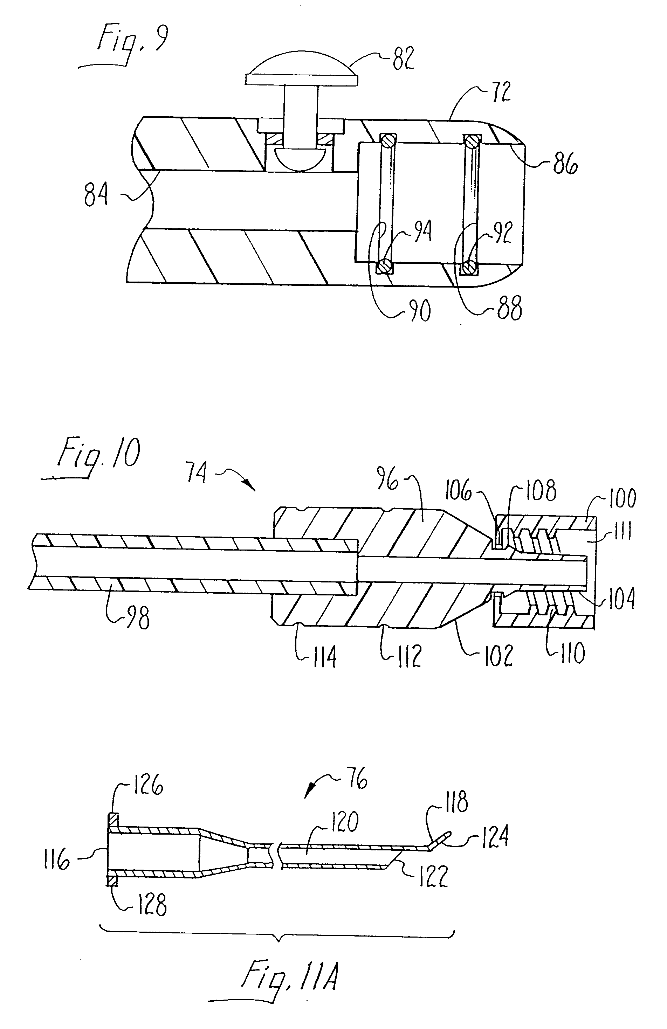

Referring now to Fig. 8, the connection between handle 72 and probe tip 76 via probe connecter 74 is shown. In order to better understand the interaction of the various components, cross-referencing Figs. 9 through 11B is helpful. Figs. 9 through 11B show handle 72, probe connector 74, and a probe tip 76, respectively, in isolation.

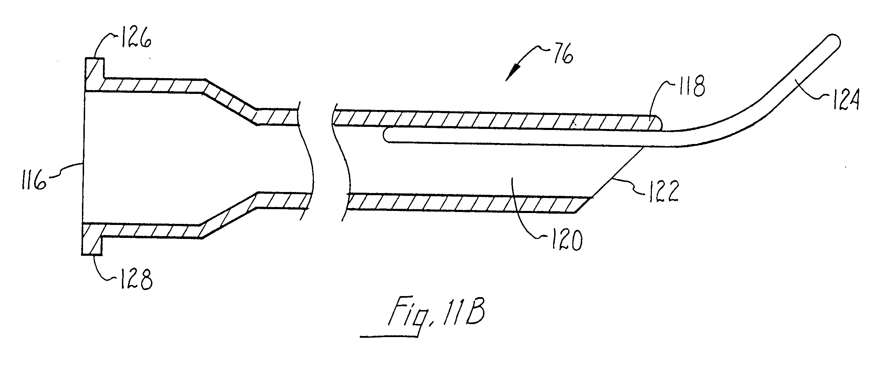

Referring now to Fig. 9, handle 72 is shown. As is apparent, handle 72 includes a flow regulator 82 similar to regulator 40 shown in Figs. 4 and 6A above. Handle 72 is formed with a lumen, or longitudinal channel 84 which extends from the proximal end of the handle to recess 86 at the distal end of handle 72. Recess 86 is preferably cylindrical and includes one or more, preferably two, annular grooves 88 and 90, into which o-rings 92 and 94 are placed. Recess 86 is sized to receive probe connector 74, shown in Fig. 10.

Referring now to Fig. 10, probe connector 74 is shown. Connector 74 includes body portion 96 which is preferably cylindrical, flow control tube 98 and threading collar 100. Flow control tube 98 is attached to the proximal end of body 96. Preferably, tube 98 is inserted into the proximal end of body 96 to ensure a firm connection. The distal end of body 96 includes a tapered portion 102 where the body 96 narrows to tip 104.

Tip 104 is surrounded by threading collar 100. Collar 100 is able to rotate relative to body 96, but is held to body 96 by the cooperation between inwardly facing annular shoulder 106 and outwardly facing annular ridge 108. The inner surface of collar 100 includes threads 110. Collar 100 is sized to leave an annular space 111 between the collar 100 and tip 104 of the probe connector 74.

Body 96 includes, on its outer surface, one or more, preferably two, annular grooves 112 and 114 which can receive the o-rings 92 and 94 shown in Fig. 9.

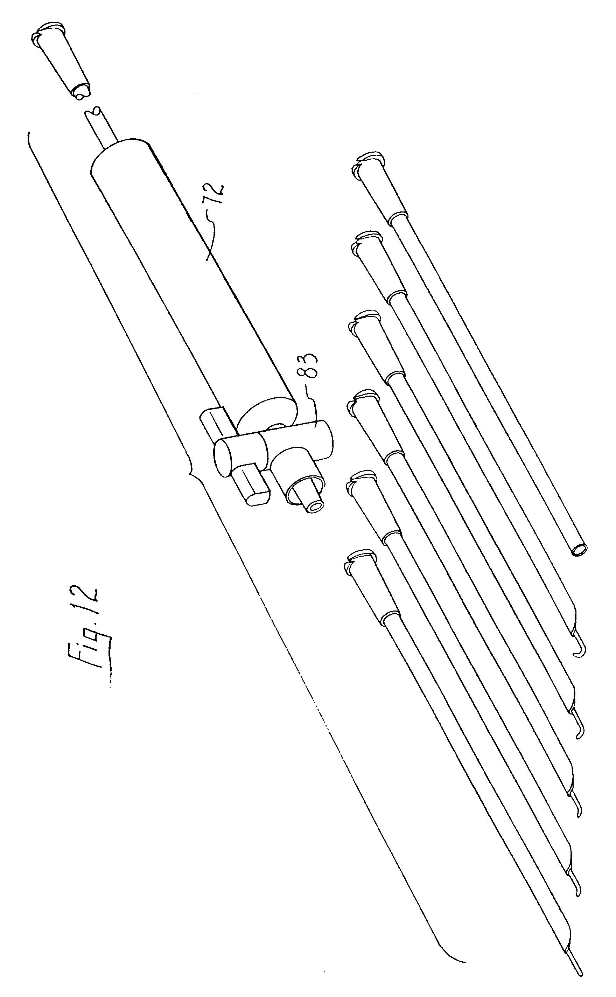

Referring now to Figs. 11A and 11B, probe tip 76 has a proximal end 116 and a distal end 118. Generally, probe tip 76 is an elongated tube forming a fluid passageway 120. The distal end 118 includes a fluid opening 122 through which irrigating fluids may flow.

A tongue member 124 is attached to the probe tip 76 adjacent opening 122. Figs. 11A and 11B show two different methods of placing tongue member 124 on probe tip 76. Tongue 124 is shaped to facilitate probing during irrigation operations. As those skilled in the art will appreciate, alternate tips may be desirable for various probing/irrigation operations. The distal end 118 can be replaced by a variety of tip configurations, including those shown in Figs. 5A and 5B without departing from the scope of the present invention.

The proximal end 116 of probe tip 76 is formed to cooperatively engage the distal end of the probe connector 74. More specifically, probe end 116 is sized to fit into annular space 111 between collar 100 and connector tip 104 (shown in Fig. 10). Typically, probe end 116 is enlarged to fit over connector tip 104. Additionally, probe tip 76 has, near its proximal end 116, one or more, preferably two, lugs 126 and 128. These lugs 126 and 128 extend outwardly and are sized to cooperatively engage the threads 110 of collar 100.

Referring again to Fig. 7, a fluid line connector 78 is shown inserted into the proximal end of handle 72. Connector 78 is shown as a connector onto which the female end of a fluid line 80 can be attached. It is likewise possible to use any other fluid connector, including a Luer lock, without departing from the scope of the invention.

Detachable instrument 70 is assembled by inserting probe connector 74 into recess 86 of handle 72 until o-rings 92 and 94 engage grooves 112 and 114, thereby establishing a fluid seal between the handle 72 and the probe connector 74. Next, end 116 of probe tip 76 is inserted into annular space 111 between collar 100 and connector tip 104. Collar 100 is rotated until lugs 126 and 128 engage threads 110. Collar 100 is then rotated to draw in probe tip 76 until end 116 contacts connector body 96, thereby forming a fluid seal between the connector body 96 and probe tip 76. The handle 72, probe connector 74, and probe tip 76 are shown in the assembled configuration in Fig. 8. Finally, a connector 78 is then attached to the proximal end of the handle 72, onto which a fluid supply line 80 can be attached to achieve the configuration shown in Fig. 7.

The probe of this invention can be provided with interchangeable probe tips to facilitate changing probe configurations or sizes, as shown in the alternate embodiment in Fig. 12. Also shown in Fig. 12 is an alternate embodiment of a flow regulator which is simply a plug valve 83 or other valve known in the art, installed in a bore through the handle 72, eliminating the flow control tube.

### OPERATION

Operation of the present invention will be discussed in terms of the embodiment shown in Figs. 3 through 6B, although it is to be appreciated that the operation of the embodiment shown in Figs. 7 through 11B is substantially the same.

Operation of the irrigation probe instrument 10 is initiated by connecting instrument 10 to a fluid line 18 via connector 16. After connection, the probe tip 26 can be inserted into the surgery site. The surgeon, by manipulating the instrument 10, positions the distal end 30 to move obstructing tissue or to position the openings 36, 36a, 36b for lavaging selected locations.

To restrict the flow of irrigating fluid through the instrument 10 and out of the fluid line 18, the surgeon applies pressure on button 50, 60 which slides restrictor 44, 56 inwardly. As restrictor 44, 56 moves inwardly, it engages flow control tube 38. As the restrictor continues to move inwardly, flow control tube 38 is deformed, resulting in a narrowing 62 in flow control tube 38. This narrowing 62 restricts flow through tube 38 and can, if great enough, cut off flow altogether.

To resume irrigating, the surgeon merely releases the button 50, 60. As a result of the pressure inside the joint, the narrowing 62 is forced outwardly, and unrestricted flow is possible. Alternately, using the embodiment shown in Fig. 12, the surgeon can adjust the plug valve 83 to increase or decrease flow.

While the particular Irrigation Probe Assembly as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown, other than as described in the appended claims.

## Claims

1. A device for probing and irrigating the interior of a being, said device comprising:
a handle having a fluid passageway;
a hollow irrigation probe tip formed with a lumen and having a proximal and a distal end, said proximal end of said irrigation probe tip being connectable to said fluid passageway, and said distal end of said probe tip being shaped to enter and probe the interior of the being;
at least one opening in said irrigation probe tip for allowing flow of the fluid through said probe tip; and
flow regulation means attached to said handle for regulating flow of the fluid through said fluid passageway.

2. The device according to claim 1 wherein said handle is formed with a longitudinal bore therethrough and said fluid passageway is at least partially located in said longitudinal bore of said handle.

3. The device according to claim 1 or 2 wherein said distal end of said irrigation probe tip is bent to facilitate probing.

4. The device according to any one of claims 1 to 3 wherein said opening in said probe tip is an opening at an extremity of said tip, with said opening allowing the fluid in said lumen to flow through said probe tip at said extremity.

5. The device according to claim 1 wherein said distal end of said probe tip forms a solid extremity and said opening in said probe tip is a lateral opening through a portion of said probe tip other than said extremity, said lateral opening allowing the fluid in said lumen to flow through said probe tip.

6. The device according to any one the preceding claims wherein said flow regulating means comprises a valve.

7. The device according to any one of the preceding claims further comprising a flow tube through said fluid passageway wherein said flow regulating means selectively collapses said flow tube to restrict flow of the fluid through said flow tube.

8. The device according to claim 7 wherein said flow regulating means comprises a flow restrictor slidably retained in a recess in said handle.

9. The device according to claim 8 wherein said flow restrictor is attached to a button allowing an operator of said device to collapse said flow tube and restrict fluid flow through said fluid passageway.

10. The device according to claim 9 wherein said flow restrictor is formed with an annular ridge, said annular ridge cooperatively engaging an annular retainer in said recess to retain said flow restrictor in said recess.

11. The device according to claim 9 wherein said flow restrictor includes a threaded portion and said recess is formed with a threaded flange through which said threaded portion of said flow restrictor is threaded, said flow restrictor being slidably retained in said recess by cooperative engagement between said threaded portion and said flange.

12. The device according to any one of the preceding claims wherein said probe tip is attachable to a probe connector at least partially inserted into said handle.

13. The device according to claim 12 wherein said proximal end of said probe tip is threaded into a collar rotatably attached to said probe connector, said collar cooperatively maintaining said probe tip in contact with said probe connector to establish a seal between said probe tip and said probe connector.

14. The device according to claim 13 further comprising at least one o-ring between said handle and said probe connector to establish a seal therebetween.

15. A device according to any one of the preceding claims and further comprising:
an irrigating fluid source;
a pump in fluid communication with said source; and
a fluid supply line in fluid communication with said pump, the irrigating fluid being moved through said fluid supply line into the interior of the being by said pump.

16. The device according to claim 15, wherein said fluid supply line supplies irrigation fluid through a separate entry point into the interior of the being and the irrigation fluid is drained from the interior of the being through said irrigation probe tip.

17. The device according to claim 15, wherein said fluid supply line supplies irrigation fluid through said irrigation probe tip into the interior of the being.
